# EUROPEAN PATENT SPECIFICATION

(11) **EP 0 571 491 B1**
(45) Date of publication and mention of the grant of the patent: **29.01.1997**
(21) Application number: 92905459.1
(22) Date of filing: 14.02.1992
(51) Int. Cl.: A61K 31/35, C07D 311/32

(54) **BENZOPYRAN PHENOL DERIVATES FOR USE AS IMMUNOSTIMULATING AGENTS**
BENZOPYRANPHENOLDERIVATE ZUR VERWENDUNG ALS IMMUNSTIMULIERENDE MITTEL
DERIVES PHENOLIQUES DE BENZOPYRANNE DESTINES A ETRE UTILISES EN TANT QU'AGENTS IMMUNOSTIMULANTS

(30) Priority: 15.02.1991 US 656801; 17.12.1991 US 809420
(43) Date of publication of application: 01.12.1993
(60) Divisional application: 95116380.7
(73) Proprietor: Fockerman, Jasmine, S-260 70 Ljungbyhed (SE); Fockerman, Michel, S-114 54 Stockholm (SE)
(72) Inventor: MARCONIUS, Maria, S-121 74 Johanneshov (SE)
(74) Representative: Fagerlin, Heléne
(86) International application number: PCT/SE92/00090
(87) International publication number: WO 92/14458

(56) References cited:
- EP-A- 0 310 757
- EP-A- 0 352 147
- FR-A- 2 209 574
- STN International File CA, CHEMICAL ABSTRACTS, Vol. 110, No. 23, 5 June 1989, Columbus, Ohio, US; BANKOVA V. et al.: "On the chemical composition of some propolis fractions with antiviral action", Acta Microbiol. Bulg. 23, 52-7
- CHEMICAL ABSTRACTS, Volume 91, No. 11, 10 September 1979, Columbus, Ohio, US; METZNER J. et al.: "Antimicrobial activity of propolis and propolis constituents", see page 141, Abstract 84014x
- CHEMICAL ABSTRACTS, Volume 101, No. 9, 27 August 1984, Columbus, Ohio, US; BOGDANOV S. et al.: "Characterization of antibacterial substances in honey", see page 534, Abstract 71363k
- PATENT ABSTRACTS OF JAPAN, Vol. 13, No. 273, C609; & JP-A-01 068 319
- Die Pharmazie, 34 (1979), 97-102

## Description

The benzopyran phenol derivates used according to the invention can be derived from propolis be ethanol extraction and are used as an immunostimulating agent.

Propolis, also known as bee glue, is a natural product of bees. Bees collect propolis on the buds and other parts of plants, using it in their habitat to block up holes and cracks and also to isolate foreign bodies (insects and other living creatures) in the hive, thus preventing the spread of infections. It is also used by the bees to coat the cells of the honeycomb before storing their products such as honey and pollen in the honeycomb cells.

Propolis is a specific complex bioactive substance consisting of more than 60 compounds. The basic components are different resins, waxes, ethereal oils and pollen. In addition, major bioactive ingredients of propolis are vegetable dyes, of which the most important are the flavones or yellow dyes such as chrysin or tectochrysin, flavonones such as pinostrombin, pinocembrin and quercetin and their derivatives, and also flavonols such as rhamnocitrin, galangin and isoalpinin. It also contains aroma substances such as isovanillin and acetoxybetulinol, and aromatic acids such as cinnamic acid, benzoic acid, caffeic acid, ferulic acid and protocatechuic acid with their esters with benzyl alcohol, pentanol, phenylethyl alcohol and cinnamyl alcohol Die Pharmazie No. 34, 1979, 103 and Die Pharmazie No. 34, H.2, 1979, 97-102).

It has now turned out that the compounds with the general formula I 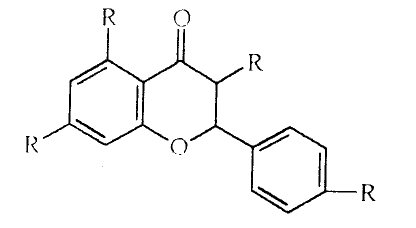 in which R is the same or different and represents H, OH or 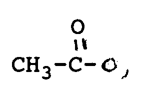 are components in propolis that can be used as immuno-stimulating agents, which agents have better effects than propolis.

The invention concerns the use defined in the claims and especially concerns the use of Pinocembrin, 4H-1-benzopyran-4-one, 2,3-dihydro-5,7-dihydroxy-2-phenyl, Pinobanksin-3-acetate, 4H-1-benzopyran-4-one, 2,3-dihydro-5,7-dihydroxy-2-phenyl-3-acetate and Naringenin, 5,7,4'-trihydroxy-2,3-dihydro-2-phenyl-1,4-benzopyron or 5,7,4'-flavanone for preparing a composition for use as an immuno-stimulating agent.

The invention also concerns the use of mixtures of compounds with the general formula I and especially mixtures of Pinocembrin, and Pinobanksin- 3-acetate and Naringenin. Preferably the three compounds are used together in the treatment. The extract produced according to the invention is called Propinocom, shortened to PRP-C.

The mixture can be prepared by a process where a propolis product is extracted with water or a water solution containing a salt, preferably a pharmaceutical acceptable salt. When the inventor first made such an extract she used a 0.9 weight % NaCl solution (physiological sodium chloride solution) with the intention for human or veterinary use. It has, however, turned out that water may be used and that the extraction times may be shortened when pure water is used. Thus water extraction is preferred. A salt solution may, however, be used and any salt is suitable. Preferably a pharmaceutically acceptable salt is used, such as NaCl or KCl. When water is used the product may be freeze dried. When a salt solution is used it may be dialyzed first to take away the salt.

The mixture used in accordance with the invention is obtainable by adding the propolis product preferably in an organic solvent such as an alcoholic solution to water or a water solution containing 0.1-17 weight % of NaCl with a temperature of 30-95°C, and keeping the mixture at 30-95°C for 10 to 100 hours, whereafter the solution is freed from the bottom sediment.

The alcohol may be an alcohol containing 1-20 carbon atoms, preferably 1-5 carbon atoms, such as methanol, ethanol, propanol, butanol, especially ethanol. Preferably there is used an ethanolic propolis solution prepared according to EP 0 310 757.

According to this document, an ethanolic propolis product can be prepared by extracting propolis in a closed system at a low temperature, 0-20°C with an ethanol/water mixture at a volume ratio of 87 : 13 under ultrasonic treatment from 18 to 25 kHz for a short period such as 25 minutes, the resultant suspension being decanted and the clear propolis extract freed from the solid particles. Preferably, the propolis is ground to a particle size of 3 mm diameter before extraction. The propolis is ground in a suitable mill into fine particles (max. diameter 5 mm).

The alcoholic solution is preferably added to the water or salt solution in an amount of 1-60 % by volume counted on the sum of the volumes of the two solutions.

The propolis solution is preferably added to the water or salt solution drop by drop over about 1-7 hours, preferably 5 hours. A brown sediment is formed. The mixture is kept at 30-95°C for another 5-100 hours, preferably about 40-80 hours. A temperature of 50-70°C is preferred, especially 60°C. A light yellow solution is formed over a hard dark brown sediment. The propolis solution being alcoholic is evaporated during the process and the yellow extract that is obtained has about the same volume as the NaCl solution used.

The substances and the compositions used according to the invention can be used as immunostimulating agents, such as adjuvants, and in vaccines.

By the extraction process there is obtained a product containing Pinocembrin, Pinobanksin-3-acetate, and Naringenin. The extract can be adjusted to contain preferably a physiological solution of 0.6-0.9, preferably 0.9 % NaCl.

The immunostimulating effect of the mixture used according to the invention has been tested directly on cells from thymus and spleen (Band T-lymphocytes and natural killer cells (NK cells)). In these tests the mixture showed potent immunostimulating effects. The mixture has also been given to animals in their feed giving rise to increased activity of Tand B-cells in both thymus and spleen. The mixture increases both the humoral and cell mediated immune response.

In mice infected with Coxsackievirus B3 (CB3) the lifetime was significantly prolonged for mice treated prophylactically with the composition.

When tested as 1-10 % nose spray in a total of 58 patients no local or systemic allergic reactions were observed.

The substances and the compositions used according to the invention can be used in any pharmaceutical form such as tablets, capsules, solutions for injections, solutions or spray for nasal treatment.

The administration forms may contain pharmaceutically acceptable carriers such as one or more compatible solid filler diluents or solid or liquid substances added to aid in the production of the pharmaceutical forms, such as lubricants to reduce friction and glidants to improve flow of the particulate mixtures. By "compatible" as used herein, is meant that the components are capable of being comingled without interacting in a manner which would substantially decrease the pharmaceutical efficacy.

Some examples of substances which can serve as pharmaceutical carriers are sugars, such as lactose, glucose and sucrose; starches, such as corn starch and potato starch; cellulose and its deivatives, such as sodium carboxymethylcellulose, ethylcellulose, cellulose acetate; powdered tragacanth; malt; gelatin; talc; stearic acid; magnesium stearate; zinc stearate; calcium sulphate; silicon dioxide; vegetable oils, such as peanut oil, cottonseed oil, sesame oil, olive oil, corn oil and oil of theobroma; polyols, such as propylene glycol, glycerine, sorbitol, mannitol, and polyethylene glycol; agar; and alginic acid; as well as other non-toxic compatible substances used in pharmaceutical formulations. Wetting agents such as sodium laural sulphate, as well as coloring agents, lubricants, excipients, stabilizers, antioxidants, and preservatives, can also be present.

For nasal treatment there can be used a physiological NaCl-solution containing 0.9 % by weight NaCl and 1-20 % propolis extract.

### BRIEF DESCRIPTION OF THE DRAWINGS

Figure 1 shows the immune cell activity of T-(Con A) in spleen and thymus and B-cells (LPS) in spleen after propolis treatment in vivo.
Figure 2 shows the survival of Coxsackievirus B3 in untreated and propolis treated Balb/c mice.

The preparation of the mixture and features and advantages of the present invention will be more clearly understood by reference to the following examples.

### Example 1 (Preparation of the starting material)

In the extraction vessel, 500 litres of an ethanol solution not less than 87 % by weight in water is prepared. While the mixture is being stirred, 20 kg of the ground propolis is added to the contents of the extractor. The batch is subjected to ultrasonic extraction (18 - 25 kHz) for 25 minutes, then left to stand for 2 hours, decanted and filtered.

The filtration is effected via a pressure filter with rapid filter-paper inserts. The clear propolis extract obtained is reduced to the required concentration of 5 to 80 % of the dry substance in a column concentrator in a 150 - 50 mm H₂O vacuum by means of a heat pump. The concentration is performed at a temperature of max. 20°C.

### Example 2

The extract obtained in example 1 was diluted to contain a dry substance weight of 10 % in ethanol. NaCl was dissolved in distilled water to a concentration of 0.9 %. 700 ml of the solution was placed in a beaker which was placed into a water bath. The temperature in the beaker was kept at 30°C. 300 ml of the propolis extract con-taining 10 weight % propolis in ethanol was added drop by drop to the NaCl colution during about 5 hours. A muddy brown precipitate was obtained. The temperature of the solution was controlled by keeping the water bath boiling and by continuously adding water during 15 hours to keep the level. By then a light yellow solution was formed the volume of which was 700 ml. The rest of the solution had evapo-rated during the heating. The beaker was left in the water bath until the content had reached room temperature. The precipitate was freed from the solution. Analysis by mass spectra of the yellow solution shows that it contains Pinocembrin and Pinobanksin-3-acetate.

### Example 3

The extract obtained in Example 1 was diluted to contain a dry substance weight of 10 % in ethanol. NaCl was dissolved in distilled water to a concentration of 10 %. 700 ml of the solution was placed in a beaker which was placed into a water bath. The temperature in the beaker was kept at 30°C. 300 ml of the propolis extract containing 10 weight % propolis in ethanol was added drop by drop to the NaCl colution during about 5 hours. A muddy brown precipitate was obtained. The temperature of the solution was controlled by keeping the water bath boiling and by continuously adding water during 15 hours to keep the water level constant. A light yellow solution having a volume of about 700 ml was formed. The rest of the solution had evaporated during the heating. The beaker containing the solution was left in the water bath until the content had reached room temperature. The precipitate was freed from the solution.

### Example 4

The extract obtained in example 1 was diluted to contain a dry substance weight of 10 % in ethanol. NaCl was dissolved in distilled water to a concentration of 15 %. 700 ml of the solution was placed in a beaker which was placed into a water bath. The temperature in the beaker was kept at 30°C. 300 ml of the propolis extract containing 10 weight % propolis in ethanol was added drop by drop to the NaCl solution during about 5 hours. A muddy brown precipitate was obtained. The temperature of the solution was controlled by keeping the water bath boiling and by continuously adding water during 15 hours to keep the level. A light yellow solution having the volume of about 700 ml was formed. The rest of the solution had evaporated during the heating. The beaker containing the solution was left in the water bath until the content had reached room temperature. The precipitate was freed from the solution.

### Example 5

The procedure of example 2 was repeated but with 800 ml of the NaCl solution and 200 ml of the propolis extract. The resultant solutions were analyzed by mass spectroscopy as follows. Two ml of the yellow extract were extracted with 2 ml ethyl acetate. Volumes of 1 and 5 microliters of the ethyl acetate phase were injected into a gas chromatograph coupled to a Jeol 300 mass spectrometer. The gas chromatograph comprised a 15 m long capillary column with unpolar stationary phase. Splitless injection was made 1 minute 240°, temperature processing; 45°, 1 minute, 10° per minute. The mass spectrometry analysis parameters were EI 70 EV 1.2 seconds/scan from mass 35 to 300. The inner standard was added to the sample with 430 µl of 0.7 µg/ml of Pinocembrin-7-methyl ether before the extraction was performed.

The inner standard Pinocembrin-7-methyl ether was also present in the sample, which makes analysis difficult. In order to be able to use the inner standard, the sample was chromatographed with and without inner standard so that the interference by the inner standard may be subtracted. Pinocembrin and pinobanksin-3-acetate were identified with reference spectra.

These results show that 2 ml of the extract, which is a 0.9 % NaCl solution, contains 200 ng/ml (20 x 10⁻⁸ g/ml) of Pinocembrin and 90 g/ml (90 x 10⁹ g/ml) of Pinobanksin-3-acetate. There are no other detectable components in the extract.

### Example 6

The procedure of example 2 was repeated but there was used instead 770 ml of the NaCl solution and 230 ml of the propolis extract.

### Example 7

The procedure of example 2 was repeated but there was used instead 500 ml of the NaCl solution and 500 ml of the propolis extract.

The light yellow solution was further purified by dialysis against water. 50 ml of the extract was dialysed against 250 ml of distilled water through a tube (Model mw cataf 3500 Kebo AB Sweden).

### Example 8

The procedure of Example 7 was repeated but after adding the propolis exract drop by drop to the NaCl solution, the mixture was heated at 60°C for 76 hours. The yellow extract was separated from the precipitate and analyzed. Another dialysis equipment than in example 7 was used. Analysis of the yellow solution with mass spectra shows that it contains pinocembrin, pinobanksin-3-acetate and naringenin.

### Example 9

The procedure of Example 2 was repeated but after adding the propolis extract drop by drop to the NaCl solution, the mixture was heated at 60° for 78 hours. Another dialysis equipment than in example 7 was used. Analysis of the yellow solution with mass spectra shows that it contains pinocembrin, pinobanksin-3-acetate and naringenin.

### Example 10

The starting material was prepared as described in Example 1, and the clear propolis extract was reduced to a concentration of 20 weight %. Thereafter, the procedure of Example 7 was repeated, but after adding the propolis extract drop by drop to the NaCl solu-tion, the mixture was heated at 60° for 72 to 78 hours. Another dialysis equipment than in example 7 was used. Analysis of the yellow solution with mass spectra shows that it contains pinocembrin, pinobanksin-3-acetate and naringenin. This extract is suitable as an antiviral agent, for example, through stimulation of the immune system (see Example 15).

Examples 1 to 10 above can be repeated using a water solution instead of a NaCl solution.

### Example 11

Example 10 was repeated but there was used 500 ml water and 500 ml of 20 weight % propolis extract. The temperature was kept at 60°C and the extraction time ws 32 hours.

### Example 12

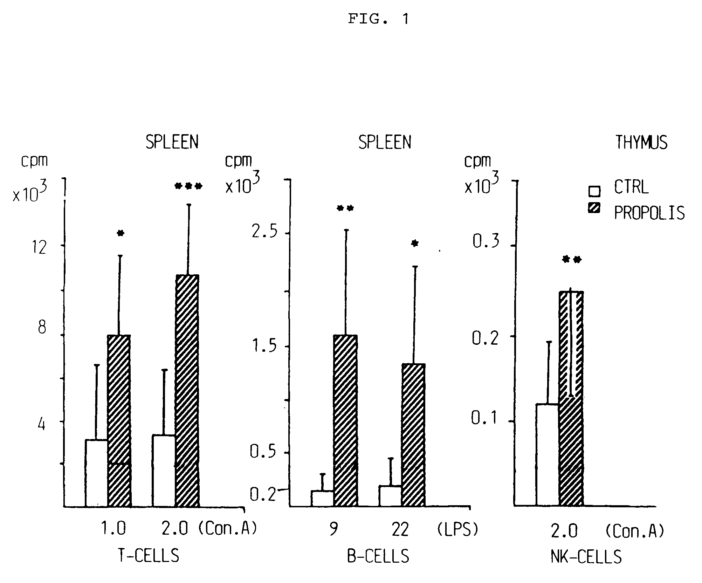

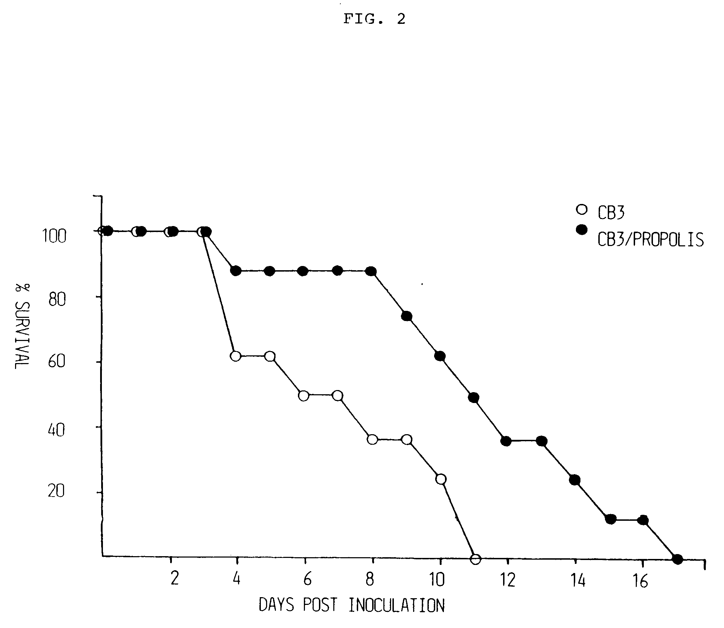

The immunostimulating effect of the mixture used according to the invention has been tested directly on cells from thymus and spleen (B- and T-lymphocytes and natural killer cells (NK cells)). In these tests, the mixture showed potent immunostimulating effects. The results of in vivo treatment are shown in Figure 1. The composition used according to the invention has also been given to animals in their feed, giving rise to increased activity of T- and B-cells in both thymus and spleen. The composition used according to the invention increases both the humoral and cell mediated immune response. In mice infected with Coxsackievirus B3 (CB3), the lifetime of the mice was significantly prolonged for mice treated prophylactically with the composition. The results are shown in Figure 2.

### Example 13

### TOXICITY IN MICE

To obtain information on the effect of the product according to the invention as an adjuvant in animals the product of example 10 was tested in mice using a dose of 1 µg.

The animals were divided into two groups, each group consisting of six female 12 weeks old BALB/c mice.

The preparation was given to two groups of animals, either as subcutaneous (s/c) or intraperitonial (i/p) injections.

### RESULTS

No lethal effect was noticed during the observation period of three weeks. But minor local reactions were observed in two out of six animals inoculated s/c. The changes observed were total depletion of the hair at the site of injection.

### Example 14

### IMMUNISATION OF MICE WITH Antigen mixed with the product of Example 10

Two groups (A and B) of 12 weeks old female BALB/c mice were used. Each group consisted of six animals.

The antigen (Ag) used for the immunisations was the envelope glycoproteins of Equine herpesvirus type-2 (EHV-2). The dose of antigen 5 µg protein for each of the two different preparations as measured by the Bradford method.

The groups of mice were immunised in the following manner:-Group (A) received the Ag mixed with Freunds incomplete adjuvant (FIA).
Group (B) received the antigen mixed with 1 µg of the product of Example 10.

The mice were immunised twice four weeks apart. Blood samples were taken at week 4, 5 and 7 after the first immunisation. Serum was separated and inactivated for 1 hour at 56°C. Serum antibody responses to the envelope antigen were measured in ELISA.

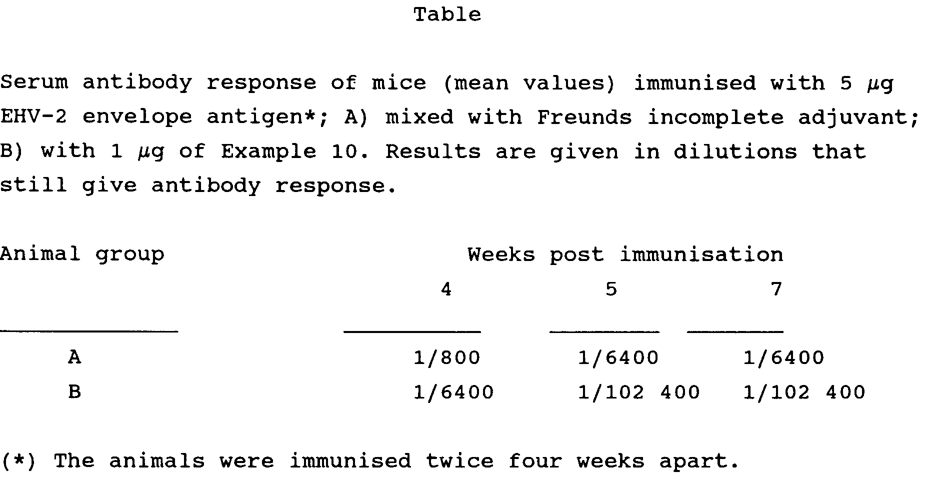

### RESULTS

Animals receiving antigen and the product of Example 10 in this test show about 16 times higher antibody titers than animals receiving antigen and FIA.

## Claims

1. Use of a mixture containing compounds according to the general formula I: 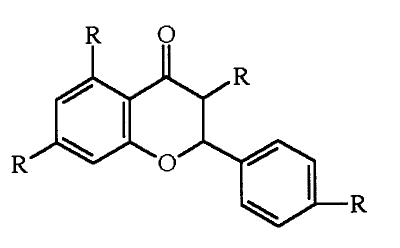 where R is the same or different and represents H-, OH-, CH₃COO-, that is obtainable by adding a propolis containing product such as an organic solvent, preferably an alcoholic solution containing propolis, to a water solution containing 0.1-17 weight % of NaCl with a temperature of 30-95 °C for 10-100 hours, whereafter the solution is freed from the bottom sediment for preparing a composition for use as an immunostimulating agent.

2. Use according to claim 1 of a mixture comprising pinobanksin-3-acetate, pinocembrin and naringenin.

## Patentansprüche

1. Verwendung elner Mischung, die Verbindungen mit der allgemeinen Formel I enthält: 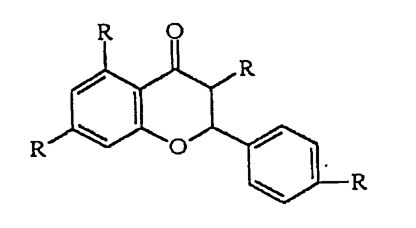 worin R gleich oder verschieden ist und H-, OH-, CH₃COO-bedeutet, welche erhältlich ist durch Zugabe eines Propolis enthaltenden Produkts, wie z.B. einem organischen Lösungsmittel, vorzugsweise einer alkoholischen Lösung, die Propolis enthält, zu einer Wasserlösung, die 0,1 bis 17 Gew.-% NaCl enthält, bei einer Temperatur von 30 bis 95°C für 10 bis 100 Stunden, worauf die Lösung vom Bodensediment befreit wird, um eine Zusammensetzung zur Verwendung als immunstimulierendes Mittel herzustellen.

2. Verwendung einer Mischung nach Anspruch 1, die Pinobanksin-3-acetat, Pinocembrin und Naringenin enthält.

## Revendications

1. Utilisation d'un mélange contenant des composés selon la formule générale I: 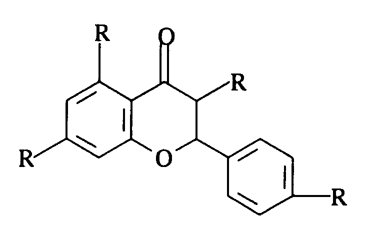 dans laquelle les symboles R sont identiques ou différents et représentent H-, OH-, CH₃COO-, qui peut être obtenu par addition d'un produit contenant de la propolis tel qu'un solvant organique, de préférence une solution alcoolique contenant de la propolis, à une solution aqueuse contenant 0,1-17 % en masse de NaCl à une température de 30-95°C pendant 10-100 h, après quoi la solution est débarrassée du sédiment inférieur pour préparer une composition destinée à être utilisée comme agent immunostimulant.

2. Utilisation selon la revendication 1 d'un mélange comprenant du 3-acétate de pinobanksine, de la pinocembrine et de la naringénine.
